# EUROPEAN PATENT SPECIFICATION

(11) **EP 1 843 997 B1**
(45) Date of publication and mention of the grant of the patent: **03.09.2008**
(21) Application number: 06720003.0
(22) Date of filing: 01.02.2006
(51) Int. Cl.: C07C 45/85, C07C 45/79, C07C 47/19

(54) **TREATMENT OF AN AQUEOUS MIXTURE CONTAINING AN ALKYLENE OXIDE WITH AN ION EXCHANGE RESIN**
VERARBEITUNG EINER WÄSSRIGEN MISCHUNG MIT EINEM ALKYLENOXID MIT EINEM IONENAUSTAUSCHHARZ
TRAITEMENT D'UN MELANGE AQUEUX CONTENANT UN OXYDE D'ALKYLENE AVEC UNE RESINE D'ECHANGE IONIQUE

(30) Priority: 03.02.2005 US 649643 P
(43) Date of publication of application: 17.10.2007
(73) Proprietor: Shell Internationale Research Maatschappij B.V., 2596 HR Den Haag (NL)
(72) Inventor: KOMPLIN, Glenn Charles, Katy, , Texas 77494 (US); POWELL, Joseph Broun, Houston, Texas 77070 (US); WEIDER, Paul Richard, Houston, Texas 77083 (US)
(74) Representative: Zeestraten, Albertus W. J.
(86) International application number: PCT/US2006/003441
(87) International publication number: WO 2006/083896

(56) References cited:
- US-A- 5 986 145
- DATABASE COMPENDEX [Online] ENGINEERING INFORMATION, INC., NEW YORK, NY, US; POPOOLA A V: "Mechanism of the reaction involving the formation of dioxane byproduct during the production of poly (ethylene terephthalate)" XP002387209 Database accession no. EIX92051163317 cited in the application & J APPL POLYM SCI; JOURNAL OF APPLIED POLYMER SCIENCE NOV 20 1991, vol. 43, no. 10, 20 November 1991 (1991-11-20), pages 1875-1877,

## Description

### Field of the Invention

This invention relates to a method of treating an aqueous mixture containing a water soluble alkylene oxide with an ion exchange resin without attendant production of dioxane.

### Background of the Invention

1,3-propanediol (PDO) is an industrially important chemical. PDO is used as a monomer unit to form polymers such as poly (trimethylene terephthalate) that are used in the production of carpets and textiles. PDO is also useful as an engine coolant, particularly in cooling systems that require coolants having low conductivity and low corrosivity.

PDO can be prepared in a two-step process in which ethylene oxide is first hydroformylated in an organic solution in the presence of a metal catalyst such as a cobalt or rhodium carbonyl to form 3-hydroxypropionaldehyde (HPA). The HPA intermediate is water extracted from the organic phase under pressure and the metal catalyst is recycled to the hydroformylation reaction in the organic phase. In the second step, the aqueous HPA is hydrogenated to PDO.

Ideally, the aqueous HPA extract could be routed directly to a hydrogenation reactor. However, carbon monoxide dissolved in the water is a poison for most heterogeneous hydrogenation catalysts, as is the small amount of metal from the hydroformylation catalyst that typically leaches into the water phase during extraction of HPA. For acceptable hydrogenation product yields, the metal from the hydroformylation catalyst and carbon monoxide must be removed from the aqueous HPA solution under conditions that do not degrade HPA.

U.S. Patent No. 5,986,145 (the '145 patent) provides a method for purifying an aqueous solution of HPA containing carbon monoxide and cobalt and/or rhodium compounds from a hydroformylation catalyst to remove the carbon monoxide and the cobalt and/or rhodium from the aqueous solution so the HPA in the aqueous solution can be hydrogenated to PDO. An aqueous solution of HPA is obtained by extracting an ethylene oxide-syngas hydroformylation reaction mixture with water and separating the aqueous extract. The separated aqueous extract is 1) degassed to off-gas carbon monoxide from the solution by reducing the pressure on the solution; and 2) contacted with oxygen under acidic conditions at a temperature of 5°C to 45°C to oxidize cobalt and/or rhodium in the solution and strip carbon monoxide from the solution. The aqueous solution is preferably contacted with oxygen by sparging the aqueous solution with air. The degassed and oxygen-treated aqueous solution typically contains HPA and one or more water soluble cobalt, or rhodium species. The degassed, oxygen-treated aqueous solution is placed in contact with an acidic ion exchange resin maintained at a temperature of less than 45°C to remove the soluble metal species from the solution. A strong (sulfonic) acid resin in its acid form is preferred to remove the soluble metal species since the acid form of a strong acid resin strongly adsorbs oxidized metal species such as cobalt, and is readily regenerated in a single step with sulfuric acid. After the aqueous solution is separated from the ion exchange resin, the solution may be hydrogenated to hydrogenate HPA in the solution to PDO.

The method of then '145 patent, however, has been found to produce 1,4-dioxane as a byproduct when a strong (sulfonic) acid resin is used to remove the water soluble metal species from the aqueous solution of HPA. The aqueous HPA solution extracted from the hydroformylation reaction also contains ethylene oxide, a hydroformlyation reactant, as well as HPA, and small amounts of metal compounds derived from the hydroformylation catalyst. Ethylene oxide binds to the strong (sulfonic) acid resin along with the soluble metal species from the hydroformylation catalyst when the aqueous HPA solution is contacted with the resin, forming ester bonds with sulfonic acid. Although ethylene oxide binds to the resin much more slowly than the soluble metal species, ethylene oxide binding significantly impairs the efficiency of the resin over time by fouling active sites on the resin that could bind the soluble metal species but for the ethylene oxide bound to the sites. Over time, ethylene oxide fouling reduces ion exchange capacity to near zero, if not reversed.

The ethylene oxide and the metal species can be removed from the strong (sulfonic) acid resin by regenerating the resin with a hot acid wash. The acid wash probably removes ethylene oxide from the resin by degrading esters formed between the ethylene oxide and the resin.

One of the degradation products formed is 1,4-dioxane. 1,4-dioxane is a probable human carcinogen and a non-biodegradable pollutant that is very difficult to separate from the spent acid regenerant stream-it cannot be efficiently separated from an aqueous solution by low cost technologies such as distillation or stripping. The presence of 1,4-dioxane as a byproduct of the process of producing PDO, therefore, is undesirable.

### Summary of the Invention

In one aspect, the present invention provides a process of treating an aqueous mixture, comprising contacting an aqueous mixture containing water soluble ethylene oxide with a carboxylic acid ion exchange resin at a pH of from 2.0 to 5.0 (as measured at a temperature of from 5°C to 45°C) to form an aqueous product; separating the aqueous product from the carboxylic acid ion exchange resin; after separating the aqueous product from the carboxylic acid ion exchange resin, contacting an acid wash with the resin at a temperature of at least 60°C to regenerate the resin and form a dioxane-free spent acid wash; and separating the dioxane-free spent acid wash from the resin. In an embodiment of the invention, the aqueous mixture contains a water soluble cation or cationic complex, and the aqueous product contains a lower concentration of the water soluble cation or cationic complex than the aqueous mixture. In another embodiment of the invention, the aqueous mixture contains an aldehyde, preferably HPA, and the aqueous product contains at least 70 mole percent of the aldehyde present in the aqueous mixture prior to contacting the aqueous mixture with the carboxylic acid ion exchange resin.

### Brief Description of the Drawings

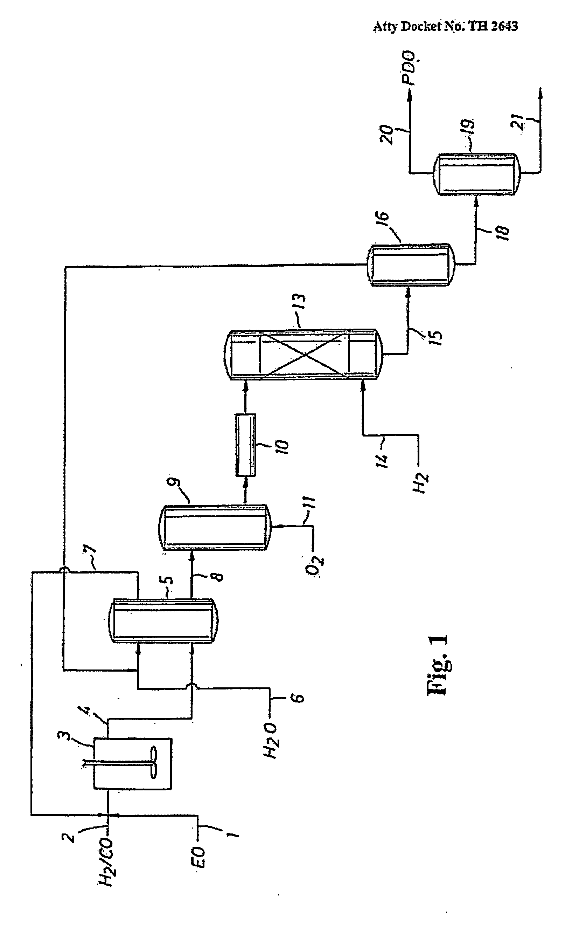

Fig. 1 is a schematic illustrating a process for removal of ethylene oxide and metal species such as cobalt or rhodium from an aqueous HPA solution in a process for preparing PDO by hydroformylation of ethylene oxide and syngas to HPA followed by hydrogenation of the HPA to PDO.

### Detailed Description of the Invention

The present invention resides in the discovery that dioxane byproduct formation in the purification of an aqueous solution containing ethylene oxide can be eliminated by using a carboxylic acid ion exchange resin to purify the aqueous solution instead of a sulfonic acid ion exchange resin. This was a very surprising discovery since it was found that dioxane formation occurred as a result of degradation of esters formed between sulfonic acid sites of a sulfonic acid ion exchange resin and ethylene oxide when regenerating the resin, and it was known in the art that dioxane forms by decomposition of a carboxylic acid ester (from a polyethylene glycol based polyester) similar to that which would be formed by esterification of a carboxylic acid site with ethylene oxide (*See* A.V. Popoola, J. Applied Polymer Sci.. 43: 1875-1877 (1991)). It was even more surprising when it was found that ethylene oxide fouled carboxylic acid ion exchange resins in a manner similar to ethylene oxide fouling of sulfonic acid ion exchange resins, indicating that ethylene oxide bound to the carboxylic acid ion exchange resin as an ester, yet did not degrade to 1,4-dioxane when regenerating the resin.

The aqueous mixture containing ethylene oxide that is to be contacted with the ion exchange resin may be any aqueous mixture containing an amount of water soluble ethylene oxide. In a preferred embodiment, the aqueous mixture is formed within a process for producing HPA from a hydroformylation reaction between ethylene oxide and syngas (carbon monoxide and hydrogen) in an organic solvent, where the aqueous mixture is formed by extracting the hydroformylation reaction mixture with water and separating the aqueous extract phase from the hydroformylation reaction mixture. Typically the aqueous mixture derived by extracting a hydroformylation reaction mixture may contain from 0.01 wt. % to 20 wt. % ethylene oxide, and more typically from 0.1 wt. % to 15 wt. % ethylene oxide.

In a preferred embodiment, the aqueous mixture also contains a water-soluble cation or cationic complex that may be adsorbed onto a carboxylic acid ion exchange resin, and thereby may be removed from the aqueous mixture. In a particularly preferred embodiment the water-soluble cation or cationic complex is one or more water-soluble metal compounds or species that may be adsorbed onto a carboxylic acid ion exchange resin. In a most particularly preferred embodiment the metal species are derived from the aqueous extraction of a hydroformylation reaction mixture containing a metal carbonylation catalyst, where the metal species in the aqueous solution are derived from the carbonylation catalyst. Preferably the metal species are cobalt and/or rhodium species, most preferably cobalt and/or rhodium cations.

In a further preferred embodiment, the aqueous mixture also contains an aldehyde. The aldehyde may be derived from the aqueous extraction of a hydroformylation reaction mixture. Most preferably the aldehyde is HPA.

The aqueous mixture may be contacted with a carboxylic acid ion exchange resin to at least partially adsorb ethylene oxide on the resin, and to at least partially remove the water soluble cation or cationic complex, if any, from the aqueous mixture. The carboxylic acid ion exchange resin may be a commercially available carboxylic acid ion exchange resin, and preferably is a commercially available acrylic acid ion exchange resin. Commercially available acrylic acid ion exchange resins useful in the process of the present invention include Dow Mac-3 acrylic acid ion exchange resin, available from the Dow Chemical Company, Liquid Separations Group, P.O. Box 1206, Midland, Michigan 48641, USA; IRC76 acrylic acid ion exchange resin, available from Rohm & Hass Company, 5000 Richmond Street, Philadelphia, Pennsylvania 19137, USA, and C140E acrylic acid ion exchange resin, available from The Purolite Company, 150 Monument Road, Bala Cynwyd, Pennsylvania 19004, USA.

The aqueous mixture may be contacted with the carboxylic acid ion exchange resin in any manner sufficient to ensure that the ethylene oxide and the water soluble cation or cationic complex, if present, in the aqueous mixture is/are exposed to contact with the resin. For example, the aqueous mixture and the resin may be contacted in a stirred mixing tank, by flow of the aqueous mixture through a fixed bed of the resin, or by passing the aqueous mixture through a chromatography column containing the resin.

Preferably, a sufficient amount of carboxylic acid ion exchange resin is provided for contact with the aqueous solution containing the alkylene oxide and water soluble cation or cationic complex to remove most, if not all, of the water soluble cation or cationic complex from the aqueous mixture. For a batch-type process contact, a sufficient amount of resin by weight of resin to weight of aqueous mixture may be from 1:5 to 1:25, and preferably from 1:10 to 1:15. For a continuous process type contact, the aqueous solution may be passed through the resin at a volume hourly space velocity (volume of aqueous solution feed per volume of resin per hour) of from 1 h⁻¹ 1 to 10 h⁻¹, preferably from 2 h⁻¹ to 5 h⁻¹.

The aqueous mixture containing the ethylene oxide, and preferably the water soluble cation or cationic complex to be removed from the mixture, may be contacted with the carboxylic acid ion exchange resin at any temperature that does not degrade the resin or does not degrade other constituents of the aqueous mixture that are to be recovered with the aqueous product upon separation of the aqueous mixture from the resin, Preferably, the aqueous mixture and the resin maybe contacted at a temperature of from 5°C to 45°C. Most preferably the aqueous mixture and the resin are contacted at ambient temperatures.

The aqueous mixture containing the ethylene oxide, and preferably the water soluble cation or cationic complex to be removed from the mixture, and most preferably an aldehyde, may have a pH of at most 5.0, or at most 4,5, or at most 4.0, more preferably from 2.0 to 5.0, or from 2.5 to 4.5 as measured at a temperature of from 5°C to 45°C. The pH is preferably maintained at the acidic conditions noted above to minimize swelling of the resin, which significantly increases above pH 5.0, and to minimize degradation of other constituents of the aqueous mixture that are to be recovered with the aqueous product separated from the resin, e.g. an aldehyde. Notably, the pH of the aqueous mixture is generally below the manufacturer's recommended pH for using the resin, which typically is from pH 5 to pH 11.

The aqueous mixture containing the ethylene oxide is preferably contacted with the carboxylic acid ion exchange resin for a period of time sufficient to expose the ethylene oxide in the solution to contact with the resin, and, if a water soluble cation or cationic complex is present in the aqueous mixture that is to be removed from the aqueous mixture by contact with the resin, the aqueous mixture is contacted with the resin for a period of time sufficient to contact the water soluble cation or cationic complex with the resin. The period of time sufficient to expose the ethylene oxide and the water soluble cation or cationic complex in the aqueous mixture to contact with the resin will vary with the apparatus used to effect contact, with the amount of resin and amount of aqueous mixture present for contact, and with the concentrations of ethylene oxide and water soluble cation or cationic complex in the aqueous mixture. Preferably the aqueous mixture containing the ethylene oxide and the water soluble cation or cationic complex is contacted with the resin for at least 1 minute, preferably from 5 minutes to 5 hours, and more preferably from 15 minutes to 2 hours.

After the aqueous mixture containing the ethylene oxide and water soluble cation or cationic complex, if any, and the carboxylic acid ion exchange resin have been contacted sufficiently to permit the ethylene oxide and the water soluble cation or cationic complex to contact the resin, the aqueous mixture is separated from the resin. The aqueous mixture may be separated from the resin in any manner effective to separate the aqueous mixture from the solid resin, which may depend on the method the aqueous mixture is contacted with the resin. For example, if the aqueous mixture is contacted with the resin in a stirred mixing tank, the aqueous mixture may be decanted from the tank and the resin vacuum filtered to remove the aqueous mixture from the resin. Alternatively, if the aqueous mixture is contacted with the resin by column chromatography, the aqueous mixture may be separated from the resin by allowing the mixture to elute through the resin, and preferably washing the aqueous mixture from the resin with an amount of water sufficient to separate the aqueous mixture from the resin.

The aqueous solution separated from the resin is an aqueous product. If the aqueous mixture contained a water soluble cation or cationic complex prior to contact with the carboxylic acid ion exchange resin the aqueous product may contain a lower concentration of the water soluble cation or cationic complex than the aqueous mixture, and preferably the aqueous product contains at most 50 ppm of the water soluble cation or cationic complex. Most preferably, if the aqueous mixture contained at least one water soluble metal species prior to contact with the carboxylic acid ion exchange resin, the aqueous product preferably contains at most 50 ppm of each of the metal species. If the aqueous mixture contained an aldehyde, preferably HPA, prior to contact with the resin, the aqueous product preferably contains at least 70 mole percent of the aldehyde present in the aqueous solution, more preferably at least 80 mole percent, and most preferably at least 90 mole percent.

After the aqueous product is separated from the carboxylic acid ion exchange resin, the resin may be contacted with an acid wash at a temperature of at least 60°C for a sufficient time to regenerate the ion exchange activity of the resin and to produce a dioxane-free spent acid wash. As used herein the term "dioxane-free" is defined as having no detectable dioxane. The ion exchange activity of the resin may be regenerated by the acid wash by removing the water soluble cation or cationic complex from the ion exchange binding sites of the resin and/or by removing esters formed by the binding of the alkylene oxide to the resin. The acid wash should have a pH below that of the pKₐ of the carboxylic acid ion exchange resin to most fully regenerate the carboxylic acid ion exchange resin. Preferably the acid wash will have a pH of 2 or below, more preferably a pH of 1 or below. The acid wash is preferably a sulfuric acid solution, most preferably a 10% sulfuric acid solution. Other acids, however, may be utilized as the acid wash, including, hydrochloric acid, phosphoric acid, or other strong mineral acids.

In order to remove the esters formed by binding of the ethylene to the resin and increase the ion exchange capacity of the resin, the carboxylic acid ion exchange resin may be contacted with the acid wash at a temperature of 64°C or greater. The ion exchange capacity caused by removal of the ethylene oxide/resin esters is regenerated by the acid wash more rapidly at higher temperatures, and preferably the resin and the acid wash are contacted at a temperature of from 70°C to 100°C, and more preferably from 80°C to 95°C.

The carboxylic acid ion exchange resin is preferably contacted with the acid wash at elevated temperatures for a sufficient time to substantially regenerate the ion exchange capacity of the resin. Treatment times of from 0.5 hour to 2 hours have been found to be sufficient to substantially regenerated the ion exchange capacity of the resin, where higher acid wash temperatures generally shorten the required treatment time.

In a most preferred embodiment, the acid wash is contacted with the carboxylic acid ion exchange resin at a temperature of from 70°C to 100°C and a pH of at most 2 for a period of from 0.5 hour to 2 hours to regenerate the resin.

In one embodiment, the ion exchange capacity of the resin maybe regenerated by removing only the water soluble cation or cationic complex, particularly metal species, that are bound to the resin unless the ion exchange capacity of the resin is substantially impaired by ethylene oxide/resin ester formation. Typically the ethylene oxide will bind with the carboxylic acid ion exchange resin much more slowly than water soluble cations or cationic complexes such as water soluble metal species, in particular, metal cations such as cobalt cations and rhodium cations. Such water soluble metal species may be removed from the resin to regenerate ion exchange capacity of the resin by washing the resin at ambient temperatures, preferably from 15°C to 30°C, with an acid wash having a. pH below the pKₐ of the carboxylic acid ion exchange resin, preferably having a pH of 2 or lower. The resin need only be washed with hot acid having a temperature of 60°C or above when the ethylene oxide/resin esters start to significantly impair the ion exchange capacity of the resin.

After the carboxylic acid ion exchange resin has had its ion exchange capacity regenerated by contact with the acid wash, either at hot or ambient temperature, the dioxane-free spent acid wash may be separated from the resin. The spent acid wash may be separated from the resin using conventional means to separate a liquid from a solid.

The separated spent acid wash, either hot or ambient temperature, is dioxane-free. Dioxane content of the acid wash may be measured by extracting the acid wash with an organic solvent such as methoxy-t-butyl ether, and measuring the dioxane content in the solvent phase by gas chromatography, then correcting the measured value to a corrected value by consideration of the ratio of the solvent to acid wash employed in the extraction, and independently measuring the partition coefficient for dioxane in the solvent and acid, wash. A partition coefficient of 0.67 may be used for correcting the measured value of 1,4-dioxane when MTBE is used as the solvent.

Referring now to Fig..1, a particularly preferred embodiment in which a homogeneous metal catalyst is separated from an aqueous mixture containing ethylene oxide in accordance with the present invention is shown. The particularly preferred embodiment is a process for forming PDO from ethylene oxide. Separate or combined streams of ethylene oxide ("EO") 1, CO and H₂ (syngas) 2 are charged to hydroformlyation vessel 3, which can be a pressure reaction vessel such as a bubble column or agitated tank, operated batchwise or in a continuous manner. The feed streams are contacted in the presence of a hydroformylation catalyst, generally a metal carbonyl preferably selected from rhodium and cobalt carbonyls. The hydroformylation catalyst will typically be present in the reaction mixture in an amount within the range of 0.01 to 1.0 wt. %, preferably from 0.05 to 0.3 wt. %, based on the weight of the hydroformylation reaction mixture. The hydrogen and carbon monoxide will generally be introduced into the reaction vessel in a molar ratio within the range of 1:2 to 8:1, preferably 1:1 to 6:1.

The hydroformylation reaction may be carried out under conditions effective to produce a hydroformylation reaction product mixture containing a major portion of HPA and a minor portion of acetaldehyde and PDO, while maintaining the level of HPA in the reaction mixture at less than 15 wt %, preferably within the range of 5 to 10 wt.% (To provide for solvents having different densities, the desired concentration of HPA in the reaction mixture can be expressed in molarity, i.e., less than 1.5M, preferably within the range of 0.5M to 1M). Generally, the cobalt-catalyzed hydroformylation reaction may be carried out at elevated temperatures less than 100°C, preferably 60°C to 90°C, and most preferably 75°C to 85°C, with rhodium-catalyzed hydroformylations on the order of about 10°C higher. The hydroformylation reaction may be generally carried out at a pressure within the range of 1 to 35 MPa, preferably (for process economics) 7 to 25 MPa, with higher pressures preferred for greater selectivity. The hydroformylation reaction may be carried out in a liquid solvent inert to the reactants. By "inert" is meant that the solvent is not consumed during the course of the reaction. In general, ideal solvents for the hydroformylation process will solubilize carbon monoxide, will be essentially non-water miscible, and will exhibit low to moderate polarity such that the HPA will be solubilized to the desired concentration of at least 5 wt % under hydroformylation conditions, while significant solvent will remain as a separate phase upon water extraction. By "essentially non-water miscible" is meant that the solvent has a solubility in water at 25°C of less than 25 wt %, so as to form a separate hydrocarbon-rich phase upon water-extraction of HPA from the hydroformylation reaction mixture. The preferred class of solvents are alcohols and ethers which can be described by the formula

R₂—O—R₁

in which R₁ is hydrogen or C₁₋₂₀ linear, branched, cyclic, or aromatic hydrocarbyl or mono-or polyalkene oxide, and R₂ is C₁₋₂₀ linear, branched, cyclic or aromatic hydrocarbyl, alkoxy or mono- or polyalkylene oxide. The most preferred hydroformylation solvents are ethers such as methyl-t-butyl ether, ethyl-t-butyl ether, diethyl ether, phenylisobutyl ether, ethoxyethyl ether, diphenyl ether, and diisopropyl ether. Blends of solvent such as tetrahydrofuran/toluene, tetrahydrofuran/heptane, and t-butylalcohol/hexane can also be used to achieve the desired solvent properties. The currently preferred solvent, because of the high yields of HPA which can be achieved under moderate reaction conditions, is methyl-t-butyl ether.

To further enhance yields under moderate reaction conditions, the hydroformylation reaction mixture will preferably include a catalyst promoter to accelerate the reaction rate. Preferred promoters include lipophilic phosphonium salts and lipophilic amines, which accelerate the rate of hydroformylation without imparting hydrophobicity (water solubility) to the active catalyst. As used herein, "lipophilic" means that the promoter tends to remain in the organic phase after extraction of HPA with water. The promoter will generally be present in an amount within the range of 0.01 to 1.0 mole per mole of metal component of the catalyst (e.g. cobalt or rhodium). The currently preferred lipophilic promoters are tetrabutylphosphonium acetate and dimethyldodecyl amine.

At low concentrations, water serves as a promoter for the formation of the desired carbonyl catalyst species. Optimum water levels for hydroformylation in methyl-t-butyl ether solvent are within the range of 1 to 2.5 wt %. An excessive amount of water, however, reduces HPA selectivity below acceptable levels and may induce formation of a second liquid phase.

Following the hydroformylation reaction, hydroformylation reaction product mixture containing HPA, the reaction solvent, PDO, the catalyst, residual syngas, ethylene oxide, and a minor amount of by-products, may be cooled and passed to extraction vessel 5 via line 4, wherein an aqueous liquid, generally water and optional miscibilizing solvent, may be added via line 6 for extraction and concentration of the HPA for the subsequent hydrogenation step.

Liquid-liquid extraction of the HPA into the water can be effected by any suitable means, such as mixer-settlers, packed or trayed extraction columns, or rotating disk contactors. The amount of water added to the hydroformylation reaction product mixture will generally be such as to provide a water-mixture ratio within the range of 1:1 to 1:20, preferably 1:5 to 1:15 by volume. Water extraction is preferably carried out at a temperature within the range of 25°C to 55°C, with a lower temperature preferred. Water extraction under 0.5 - 5 MPa carbon monoxide at 25°C to 55°C is preferred to maximize catalyst retention in the organic phase.

The organic phase containing the reaction solvent and the major portion of the catalyst can be recycled, with optional purge of heavy ends, from the extraction vessel to the hydroformylation reaction via line 7. Aqueous extract containing HPA may be passed via line 8 to degasser-stripper-oxidizer column 9 for removal of carbon monoxide and residual syngas, then through carboxylic acid ion exchange resin bed 10 for removal of residual catalyst, and on to hydrogenation zone 13. The major portions of syngas and carbon monoxide may be removed from the aqueous extract by flash distillation upon entry into the degasser-stripper-oxidizer column 9. It has been found, however, that even minor amounts of carbon monoxide remaining in the solution can interfere with the performance of the hydrogenation catalyst, and it is preferred that residual carbon monoxide is removed, as described below, prior to passage of the aqueous HPA solution to hydrogenation.

The aqueous solution of HPA will typically contain from 4 to 60 wt % HPA, typically from 20 to 40 wt % HPA, and from 10 to 400 ppm water-soluble and water-insoluble metal compounds from the catalyst, e.g. cobalt or rhodium species such as Co[Co(CO₄]₂, Co₂(CO)₈, and Rh₆(CO)₁₆. The aqueous solution of HPA may also contain ethylene oxide.

The aqueous solution of HPA is preferably contacted with oxygen under weakly acidic conditions effective for oxidation of insoluble metal compounds, e.g. water insoluble cobalt or rhodium species, to water soluble metal compounds, e.g. water soluble cobalt or rhodium species, to facilitate removal of the metal compounds in the subsequent ion exchange step. If acid is not already present as a reaction byproduct, the aqueous HPA solution can be made sufficiently acidic by addition of an organic or inorganic acid in an amount effective to produce a solution having a pH for from 3.0 to 6.0, preferably from 3.0 to 4.0. Suitable acids include C₁₋₄ organic acids. The aqueous acid is most typically produced as a byproduct of ethylene oxide hydroformylation under conditions favoring the formation of HPA.

Oxidation can be conveniently carried out by introducing an oxygen-containing gas such as air into the aqueous HPA solution. The preferred oxidation technique involves sparging air from inlet 11 in an upward direction through degasser-stripper-oxidizer column 9 as the HPA solution to be treated flows in a downward direction through the column 9. The process may be carried out at a temperature of from 5°C to 45°C and at atmospheric pressure. Residence times typically range from 1 to 15 minutes.

Use of the sparging technique for oxidation of metal species has the added effect of sweeping carbon monoxide from the aqueous solution, particularly if an inert gas such as nitrogen or carbon dioxide is introduced with the oxidation gas to prevent formation of flammable mixtures. After the oxidation step, the aqueous solution of HPA may contain from 10 ppm to 400 ppm of one or more water soluble metal species and also may contain ethylene oxide.

A stripping gas such as nitrogen may also be sparged through the aqueous solution of HPA in the degasser-stripper-oxidizer column 9 to assist in removal of carbon monoxide and syngas from the aqueous solution of HPA. The stripping gas may be sparged through the degasser-stripper-oxidizer column 9 through the same inlet 11 as the oxidizing gas, or through a separate inlet (not shown) positioned to permit the stripping gas to flow through the aqueous solution of HPA as the solution flows through the column 9.

In an embodiment of the process of the present invention, the aqueous solution of HPA containing the water-soluble metal species and ethylene oxide may be contacted with a carboxylic acid ion exchange resin to adsorb the water-soluble metal species on the ion exchange resin 10. Optimal results are achieved in commercial processes when the resin selected for removal of the metal species has a low potential for HPA degradation, can be regenerated in a single step, and adsorbs the target metal species under pH conditions that are not conducive to degradation of HPA. Commercially available preferred carboxylic acid ion exchange resins useful in the process of the invention include DowMac-3 available from Dow Chemical, macroreticular IRC76 resin available from Rohm & Haas, and gel C104E from Purolite.

The pH and the temperature of the aqueous solution of HPA and the carboxylic acid ion exchange resin may be controlled while the aqueous solution is in contact with the resin in order to minimize degradation of HPA and limit swelling of the resin. The pH may be maintained at moderately acidic conditions from pH 2.0 to 5.0, and more preferably from 3.0 to 4.5 while the aqueous solution of HPA is in contact with the ion exchange resin to 1) minimize degradation of HPA, which is increasingly degraded above pH 5.0 and is significantly degraded at pH values approaching 6.0; and 2) to minimize swelling of the resin, which significantly increases above pH 5.0. Notably, the pH of the aqueous solution of HPA is generally below the manufacturer's recommended pH for utilizing the resin, which typically is from pH 5 to pH 11. The temperature of the aqueous solution of HPA in contact with the resin may be maintained at 45°C or below to inhibit degradation of HPA, and most preferably is maintained at a temperature of from 5°C to 45°C.

The residence time of the aqueous solution of HPA in contact with the carboxylic acid ion exchange resin should be sufficient to remove the water soluble metal species from the aqueous solution, but no longer. Long residence times are to be avoided to minimize degradation of the HPA. Residence times will be somewhat longer than that required for adsorbing the metal species on a sulfonic acid ion exchange resin, since the metal species are more strongly adsorbed on a sulfonic acid ion exchange resin than on the carboxylic acid ion exchange resin used in the process of the invention. Residence times may be controlled by length of the resin bed and flow rates of the aqueous solution of HPA through the resin. Preferred carboxylic acid ion exchange resin bed lengths will be designed to provide a space velocity between 1 and 10 volumes of feed per volume of resin bed per hour.

After the aqueous solution of HPA has been contacted with the carboxylic acid ion exchange resin, the aqueous solution may be separated from the resin to form an aqueous product containing HPA. The aqueous product containing HPA is depleted in metal species, and preferably contains at most 50 ppm of each metal species present in the aqueous solution ofHPA prior to contact with the resin.

It has been found that the carboxylic acid ion exchange resin is subject to fouling by ethylene oxide in the aqueous solution of HPA. In accordance with a step in the process of the invention, after contacting the aqueous solution of HPA containing ethylene oxide and a water soluble metal species with the carboxylic acid ion exchange resin to remove the metal species from the aqueous solution ofHPA, the resin may be contacted with an acid wash at a temperature of at least 60°C to regenerate the resin by i) removing the metal species from the resin and ii) eliminating ethylene oxide derived esters from the resin. Preferably the wash is an acid wash having a pH of less than the pKₐ of the carboxylic acid of the resin, and typically a pH of 2.0 or less. Most preferably the acid wash is a 1% to 10% sulfuric acid wash, although other acids such as hydrochloric acid or phosphoric acid may be used. The acid wash preferably has a temperature of from 70°C to 100°C, and most preferably has a temperature of from 85°C to 95°C. The resin may be contacted with the wash for a period of time sufficient to remove the metal species and eliminate ethylene oxide derived esters from the resin. Treatment times of from 0.5 hour to 2 hours are generally sufficient.

The acid wash may be separated from the resin after being contacted with the resin for a sufficient amount of time to regenerate the ion exchange capacity of the resin by removing the metal species and eliminating the ethylene oxide derived esters from the resin. The separated spent acid wash contains the metal species and, importantly, contains no 1,4-dioxane. The metal species may be recovered from the dioxane-free spent acid wash in concentrated form for conversion back into the catalytic carbonyl form to improve process economics. The remaining dioxane-free spend acid wash may be processed as a waste stream without the need for any special processes to remove 1,4-dioxane.

The aqueous product containing HPA separated from the carboxylic acid ion exchange resin may then be passed to the hydrogenation zone 13 and reacted with hydrogen 14 in the presence of a hydrogenation catalyst to produce a hydrogenation product mixture 15 containing PDO. The hydrogenation catalyst is preferably a fixed-bed supported nickel catalyst, such as is available commercially as Calsicat E-475SR and R-3142 from W.R. Grace.

The hydrogenation process can be carried out in one stage or two or more sequential temperature stages. In a preferred embodiment, hydrogenation is initially carried out at a temperature within the range of 50°C to 130°C, followed by a second stage carried out at a temperature higher than that of the first stage and within the range of 70°C to 155°C, and then optionally a third stage at a temperature greater than 120°C for reversion of heavy ends to PDO. In such a process, the illustrated hydrogenation zone 13 may include a series of two or more separate reaction vessels.

Residual solvent and extractant water can be recovered by distillation of the hydrogenation product mixture 15 in column 16 and recycled to a water extraction process for further distillation (not shown) and separation and purge of light ends. PDO containing product stream 18 can be passed to a distillation column 19 for recovery of PDO 20 from heavy ends 21.

### EXAMPLE 1

The effectiveness of a carboxylic acid ion exchange resin to remove water-soluble cobalt from an aqueous solution containing HPA, water-soluble cobalt, and ethylene oxide and to be regenerated without the production of dioxane in accordance with a process of the invention was determined. An aqueous solution containing 12.7 wt. % HPA and 78 ppm cobalt was doped with excess ethylene oxide (EO) to an EO concentration of 4.8 wt. %. The aqueous solution was doped with EO to obtain a mixture that would foul a carboxylic acid ion exchange resin quickly with EO. 0.7 grams (dry) of a carboxylic acid ion exchange resin (IRC76 from Rohm & Haas) were contacted with 14-16 grams of the EO-enhanced aqueous solution in a vial sealed with a septum, and rotated on a rotating rack for 15 hours at 25°C. The solution was then separated from the resin by filtering the resin on a vacuum filter funnel, followed by water washing the resin. The separated solution, including the water washes, was then measured for cobalt by colorimetric method, which result was recorded. The resin was placed back in the vial and 6 grams of 10 wt. % sulfuric acid in water were added to regenerate the resin. The resin was regenerated in the sulfuric acid solution for 15 hours at 90°C, after which the spent acid wash was separated from the regenerated resin. This cycle (contact of an aqueous solution with resin, separation and measurement of cobalt in the separated solution, and resin regeneration) was repeated 6 times with the same charge of resin but with different charges of the aqueous solution and the sulfuric acid solution regenerant. After the final regeneration of the resin, the final sulfuric acid regeneration solution was extracted with methoxy-t-butyl ether, and the methoxy-t-butyl ether phase of the extract was measured for 1,4 dioxane by gas chromatography, assuming a partition coefficient of 0.67 between MTBE and water. The results of measuring cobalt removal and 1,4-dioxane production are shown in Table 1.

**Table 1**

| Cycle | Cobalt (ppm) | 1,4-dioxane (ppm) |
|---|---|---|
| 0 (initial) | 78 | not detected |
| 1 | 9 | not detected |
| 2 | 17.6 | not detected |
| 3 | n.a. | not detected |
| 4 | 18.3 | not detected |
| 5 | 20.6 | not detected |
| 6 | 13.2 | 0 |

The results show that with repetitive regeneration, cobalt removal stabilized at greater than 75% for the latter cycles. Improved cobalt removal may be expected for continuous flow removal, since it is known that batchwise contacting under conditions of complete backmixing will not give complete removal of an impurity (e.g. cobalt). The results also show that in regenerating the resin no 1,4-dioxane was produced due to fouling of the resin with ethylene oxide, even in the presence of excess ethylene oxide over a series of regeneration cycles.

### EXAMPLE 2

A series of experiments were conducted to determine whether 1,4-dioxane would be formed during separation of a water soluble cobalt species from an aqueous solution containing ethylene oxide over various carboxylic acid ion exchange resins and under varying subsequent acid regeneration conditions of the carboxylic acid ion exchange resins, where the process utilized in the experiments was conducted in accordance with an embodiment of the process of the present invention. An aqueous solution containing HPA and a water soluble cobalt species was produced from the hydroformylation of ethylene oxide and syngas in the presence of a cobalt carbonyl catalyst in a methoxy-t-butyl ether solvent, followed by aqueous extraction of the hydroformylation reaction mixture, and separation and air oxidation of the aqueous extract. The aqueous solution was doped with excess ethylene oxide to concentration of between 5 wt. % and 15 wt. % ethylene oxide to accelerate fouling of the ion exchange resin with ethylene oxide. Samples of the aqueous solution were contacted with a corresponding acrylic (carboxylic) acid ion exchange resin-either IRC76 from Rohm & Haas, Mac3 from Dow Chemical, or C104E from Purolite at a ratio of one part resin per 10 parts (by weight) aqueous solution—for at least 5 days (either 5, 9, 30, or 60 days), and then the liquid phase was separated from the resin. The resin from each sample was then washed with deionized water in a filter funnel and vacuum dried. Each resin sample (1 part) was then contacted with 10 parts of an aqueous mixture of sulfuric acid of varying strength, and at varying temperatures, for 15 to 18 hours to regenerate resin capacity, and the spent acid wash was separated from the regenerated resin. Dioxane concentration was assessed by extraction of the spent sulfuric acid wash with methoxy-t-butyl ether, followed by gas chromatographic analysis of the MTBE phase assuming a 0.67 partition coefficient (MTBE/water). The results are shown in Table 2.

**Table 2**

| **Sample** | **Resin** | **Resin type** | **EO soak time** | **EO wt.%** | **Regenerant % H₂SO₄** | **Regen. Temp °C** | **Total Dioxane ppm** |
|---|---|---|---|---|---|---|---|
| 1 | IRC76 | macro | 5 | 11.8 | 1 | 90 | not detected |
| 2 | Irk76 | macro | 30 | 6.8 | 10 | 90 | not detected |
| 3 | IRC76 | macro | 30 | 6.8 | 10 | 80 | not detected |
| 4 | IRC76 | macro | 60 | 5.0 | 10 | 90 | not detected |
| 5 | Mac3 | macro | 5 | 8.0 | 10 | 90 | not detected |
| 6 | Mac3 | macro | 9 | 12.7 | 10 | 60 | not detected |
| 7 | Mac3 | macro | 9 | 12.7 | 10 | 75 | not detected |
| 8 | Mac3 | macro | 9 | 12.7 | 3 | 75 | not detected |
| 9 | C104E | gel | 5 | 12.1 | 10 | 90 | not detected |
| 10 | C104E | gel | 5 | 12.1 | 10 | 90 | not detected |

The results show no detectable 1,4-dioxane formation from regeneration of the carboxylic acid ion exchange resins after treatment with an ethylene oxide rich aqueous solution containing water soluble cobalt and HPA.

### EXAMPLE 3

For comparative purposes, a series of experiments were conducted to determine whether 1,4-dioxane would be formed in separating a water soluble cobalt species from an aqueous solution containing ethylene oxide over a strong sulfonic acid ion exchange resin and subsequent acid regeneration of the sulfonic acid ion exchange resin and separation of a spent acid wash from the regenerated resin—a process not encompassed by the process of the present invention. Samples were prepared and analyzed as disclosed in Example 2 above, except that A-15, a strong sulfonic acid ion exchange resin from Rohm & Haas, was used instead of a carboxylic acid ion exchange resin. The results are shown in Table 3.

**Table 3**

| **Sample** | **Resin** | **Resin type** | **EO soak time** | **EO wt.%** | **Regenerant % H₂SO₄** | **Regen. Temp °C** | **Total Dioxane ppm** |
|---|---|---|---|---|---|---|---|
| 1 | A-15 | macro | 5 | 0 | 10 | 90 | 0 |
| 2 | A-15 | macro | 5 | 11.8 | 10 | 90 | 997 |
| 3 | A-15 | macro | 5 | 11.8 | 10 | 90 | 499 |
| 4 | A-15 | macro | 5 | 11.8 | 0 | 90 | 1943 |
| 5 | A-15 | macro | 30 | 6.8 | 10 | 90 | 1436 |
| 6 | A-15 | macro | 30 | 6.8 | 10 | 80 | 2119 |
| 7 | A-15 | macro | 60 | 5.0 | 10 | 90 | 7851 |

The results show 1,4 dioxane formation from regeneration of all sulfonic acid ion exchange resins exposed to treatment with an ethylene oxide rich aqueous solution containing water soluble cobalt and HPA.

### EXAMPLE 4

An experiment was conducted to show that carboxylic acid ion exchange resins fouled with ethylene oxide can be regenerated by an acid wash in a process in accordance with the present invention. The fresh resin capacity of acrylic acid ion exchange resins was measured by contacting a known amount of the resin with a known amount of 0.1 N KOH overnight, with the 0.1N KOH being in excess. Titration of the residual KOH with 0.1N HCl allowed assessment of the capacity of the resin. The resins were then fouled with ethylene oxide by soaking the resins in an aqueous mixture of 5 wt.% to 15 wt.% of ethylene oxide in water for a minimum of 5 days. The fouled resin capacity (remaining capacity) was then measured by recovering the resin by filtration, vacuum drying the filtered resin, and measuring the capacity of the resin as described above with respect to fresh resin. The fouled resins were then regenerated with aqueous sulfuric acid solutions of various concentrations at various temperatures for various regeneration time periods, after which each spent acid wash was separated from its respective resin. The regeneration capacity of the resins was measured by filtering the resins, vacuum drying, and measuring the capacity as described above with respect to fresh resin. The results are shown in Table 4.

**Table 4**

| **Sample** | **Fresh resin capacity (meq/g)** | **Fouled resin capacity (meq/g)** | **Regener. H₂SO₄ (wt. %)** | **Regener. temperature (°C)** | **Regener. time (hours)** | **Regenerated resin capacity (meq/g)** | **Regen. rate (meq/g/hr)** | **Regen. rate (%/hr)** |
|---|---|---|---|---|---|---|---|---|
| 1 | 10.8 | 7.2 | 1 | 90 | 24 | 8.8 | 0.07 | 1.8 |
| 2 | 10.8 | 7.2 | 10 | 90 | 24 | 11.1 | 0.16 | 4.6 |
| 3 | 10.8 | 7.2 | 10 | 80 | 24 | 9.7 | 0.10 | 2.9 |
| 4 | 10.8 | 4.9 | 10 | 60 | 4 | 5.3 | 0.09 | 1.5 |
| 5 | 10.8 | 4.9 | 3 | 75 | 4 | 5.4 | 0.12 | 2.0 |
| 6 | 10.8 | 4.9 | 10 | 75 | 4 | 5.6 | 0.17 | 2.9 |
| 7 | 10.8 | 4.9 | 10 | 90 | 4 | 5.9 | 0.25 | 4.2 |
| 8 | 10.8 | 5.4 | 10 | 60 | 24 | 7.6 | 0.09 | 1.6 |
| 9 | 10.8 | 5.4 | 10 | 90 | 24 | 8.2 | >0.12 | >2.16 |

The results show that 1) EO-fouled carboxylic acid ion exchange resin may be regenerated by contact with an aqueous sulfuric acid solution at elevated temperature; and 2) the rate of regeneration of the EO-fouled carboxylic acid ion exchange resin increases with increasing temperature, increasing sulfuric acid strength, and increasing time.

## Claims

1. A process for treating an aqueous mixture, comprising:
contacting an aqueous mixture containing water soluble ethylene oxide with a carboxylic acid ion exchange resin at a pH of from 2.0 to 5.0 (as measured at a temperature of from 5°C to 45°C) to form an aqueous product;
separating the aqueous product from the carboxylic acid ion exchange resin;
after separating the aqueous product from the carboxylicacid ion exchange resin, contacting an acid wash with the resin at a temperature of at least 60°C to regenerate the resin and form a dioxane-free spent acid wash; and
separating the dioxane-free spent acid wash from the resin.

2. The process of claim 1 wherein the aqueous mixture contains a water soluble cation or cationic complex, and the aqueous product contains a lower concentration of the water soluble cation or cationic complex than the aqueous mixture.

3. The process of claim 2 whereir. the water soluble cation or cationic complex in the aqueous mixture is a water soluble metal species.

4. The process of claim 3 wherein contacting the carboxylic acid ion exchange resin with the acid wash after separating the aqueous product from the resin removes at least a portion of the metal species from the resin such that the spent acid wash separated from the resin contains the metal species.

5. The process of any of claims 3 or 4 wherein the water soluble metal species is a cobalt cation or a rhodium cation.

6. The process of any of claims 1-5 wherein the aqueous mixture contains an aldehyde, and wherein the aqueous product separated from the carboxylic acid ion exchange resin contains at least 70 mole percent of the aldehyde present in the aqueous mixture prior to contracting the aqueous mixture with the resin.

7. The process of claim 6 wherein the aldehyde is 3-hydroxypropionaldehyde.

8. The process of claim 7 wherein the aqueous mixture is formed by oxidizing an aqueous solution of 3-hydroxypropionaldehyde, ethylene oxide, and a cobalt or rhodium carbonyl compound under acidic conditions at a temperature of 5°C to 45°C.

9. The process of any of claims 1-3 wherein the acid wash has apH less than the pKₐ of the carboxylic acid ion exchange resin.

10. The process of any of claims 1-9 wherein the acid wash is contracted with the carboxylic acid ion exchange resin at a temperature of from 70°C to 100°C for 0.5 to 2 hours at a pH of 2 or less to regenerate the resin and form the dioxane-free spent acid wash.

## Patentansprüche

1. Verfahren zur Behandlung eines wässrigen Gemisches, umfassend:
Inkontaktbringen eines wässrigen Gemisches, welches wasserlösliches Ethylenoxid enthält, mit einem Carbonsäureionenaustauscherharz bei einem pH von 2,0 bis 5,0 (gemessen bei einer Temperatur von 5°C bis 45°C) zur Ausbildung eines wässrigen Produkts;
Abtrennen des wässrigen Produkts vom Carbonsäureionenaustauscherharz;
Inkontaktbringen einer Säurewaschlösung nach dem Abtrennen des wässrigen Produkts vom Carbonsäureionenaustauscherharz mit dem Harz bei einer Temperatur von wenigstens 60°C, um das Harz zu regenerieren und eine dioxanfreie verbrauchte Säurewaschlösung auszubilden; und
Abtrennen der dioxanfreien verbrauchten Säurewaschlösung von Harz.

2. Verfahren nach Anspruch 1, wobei das wässrige Gemisch ein wasserlösliches Kation oder einen kationischen Komplex enthält, und das wässrige Produkt eine geringere Konzentration an dem wasserlöslichen Kation oder dem kationischen Komplex als das wässrige Gemisch enthält.

3. Verfahren nach Anspruch 2, wobei das wasserlösliche Kation oder der kationische Komplex im wässrigen Gemisch eine wasserlösliche Metallspezies ist.

4. Verfahren nach Anspruch 3, wobei das Inkontaktbringen des Carbonsäureionenaustauscherharzes mit der Säurewaschlösung nach dem Abtrennen des wässrigen Produkts vom Harz wenigstens einen Teil der Metallspezies aus dem Harz entfernt, sodass die verbrauchte Säurewaschlösung, welche vom Harz abgetrennt wird, die Metallspezies enthält.

5. Verfahren nach einem der Ansprüche 3 oder 4, wobei die wasserlösliche Metallspezies ein Cobaltkation oder Rhodiumkation ist.

6. Verfahren nach einem der Ansprüche 1 bis 5, wobei das wässrige Gemisch ein Aldehyd enthält, und wobei das wässrige Produkt, welches vom Carbonsäureionenaustauscherharz abgetrennt wird, wenigstens 70 Mol-% des Aldehyds enthält, welches im wässrigen Gemisch vor dem Inkontaktbringen des wässrigen Gemisches mit dem Harz vorhanden ist.

7. Verfahren nach Anspruch 6, wobei das Aldehyd 3-Hydroxypropionaldehyd ist.

8. Verfahren nach Anspruch 7, wobei das wässrige Gemisch durch Oxidieren einer wässrigen Lösung von 3-Hydroxypropionaldehyd, Ethylenoxid und einer Cobalt- oder Rhodiumcarbonylverbindung unter sauren Bedingungen bei einer Temperatur von 5°C bis 45°C gebildet wird.

9. Verfahren nach einem der Ansprüche 1 bis 3, wobei die Säurewaschlösung einen pH von weniger als den pKₐ des Carbonsäureionenaustauscherharzes besitzt.

10. Verfahren nach einem der Ansprüche 1 bis 9, wobei die Säurewaschlösung mit dem Carbonsäureionenaustauscherharz bei einer Temperatur von 70°C bis 100°C während 0,5 bis 2 Stunden bei einem pH von 2 oder weniger in Kontakt gebracht wird, um das Harz zu regenerieren und die dioxanfreie verbrauchte Säurewaschlösung auszubilden.

## Revendications

1. Procédé de traitement d'un mélange aqueux, comprenant :
la mise en contact d'un mélange aqueux contenant de l'oxyde d'éthylène hydrosoluble avec une résine échangeuse d'ions d'acide carboxylique à un pH de 2,0 à 5,0 (mesuré à une température de 5° C à 45° C) pour former un produit aqueux ;
la séparation du produit aqueux de la résine échangeuse d'ions d'acide carboxylique ;
après la séparation du produit aqueux de la résine échangeuse d'ions d'acide carboxylique, la mise en contact d'un lavage acide avec la résine à une température d'au moins 60° C pour régénérer la résine et former un lavage acide usé sans dioxane ; et
la séparation du lavage acide usé sans dioxane de la résine.

2. Procédé selon la revendication 1, dans lequel le mélange aqueux contient un cation ou complexe cationique hydrosoluble et le produit aqueux contient une concentration inférieure de cation ou de complexe cationique hydrosoluble à celle du mélange aqueux.

3. Procédé selon la revendication 2, dans lequel le cation ou complexe cationique hydrosoluble dans le mélange aqueux est une espèce de métal hydrosoluble.

4. Procédé selon la revendication 3, dans lequel la mise en contact de la résine échangeuse d'ions d'acide carboxylique avec le lavage acide après séparation du produit aqueux de la résine élimine au moins une partie de l'espèce métallique de la résine de telle sorte que le lavage acide usé séparé de la résine contienne l'espèce de métal.

5. Procédé selon l'une quelconque des revendications 3 ou 4, dans lequel l'espèce métallique hydrosoluble est un cation cobalt ou un cation rhodium.

6. Procédé selon l'une quelconque des revendications 1 à 5, dans lequel le mélange aqueux contient un aldéhyde et dans lequel le produit aqueux séparé de la résine échangeuse d'ions d'acide carboxylique contient au moins 70 % en mole de l'aldéhyde présent dans le mélange aqueux avant la mise en contact du mélange aqueux avec la résine.

7. Procédé selon la revendication 6, dans lequel l'aldéhyde est le 3-hydroxypropionaldéhyde.

8. Procédé selon la revendication 7, dans lequel le mélange aqueux est formé en oxydant une solution aqueuse de 3-hydroxypropionaldéhyde, d'oxyde d'éthylène et un composé carbonyle de cobalt ou de rhodium dans des conditions acides à une température de 5° C à 45° C.

9. Procédé selon l'une quelconque des revendications 1 à 3, dans lequel le lavage acide a un pH inférieur à pKₐ de la résine échangeuse d'ions d'acide carboxylique.

10. Procédé selon l'une quelconque des revendications 1 à 9, dans lequel le lavage acide est mis en contact avec la résine échangeuse d'ions d'acide carboxylique à une température de 70° C à 100° C pendant 0,5 à 2 heures à un pH de 2 ou inférieur pour régénérer la résine et former le lavage acide usé sans dioxane.
